# EUROPEAN PATENT APPLICATION

(11) **EP 1 178 426 A2**
(43) Date of publication of application: **06.02.2002**
(21) Application number: 01118637.6
(22) Date of filing: 02.08.2001
(51) Int. Cl.: G06F 19/00

(54) **Medical data managing system**

(30) Priority: 03.08.2000 JP 2000240355
(71) Applicant: Nidek Co., Ltd., Gamagori, Aichi (JP)
(72) Inventor: Okamoto, Keiki, Shizuoka (JP); Noda, Manabu, Gamagori, Aichi (JP)
(74) Representative: Hager, Thomas J.

(57) **Abstract**

A medical data managing system includes: recording means for recording patient data including identification data on a patient and inspection data; a display; reception input means for inputting reception data on the patient; and control means for displaying, on the display, at least part of the patient data and at least one of reception time and an elapse time from the reception time with linking each other.

## Description

### BACKGROUND OF THE INVENTION

The present invention relates to a medical data managing system for managing data on a patient.

A patient received in a hospital waits his (her) turn to be examined in a waiting room. When the patient is called by name and is informed that his (her) turn comes, he (she) enters a consulting room to be examined. As a matter of course, if a large number of patients wait for an examination, a long time is taken for waiting.

For this reason, a notebook is put on a reception desk in the hospital so that a patient writes down his (her) name during receipt. The name of a patient who has been examined (or is being examined) is erased. Thus, it is possible to know the number of patients who are waiting for the examination before a certain patient.

Moreover, a personal computer is used to sequentially display, on a display, the time that each patient is received. Thus, the hospital (a doctor and a receptionist) can grasp the waiting situation of the patients.

However, it takes a great deal of time and labor to put a notebook on a reception desk and cause a receptionist to erase the name of a patient which has been written in the notebook. Moreover, by such a method, a doctor himself (herself) which is doing an examination cannot grasp the waiting situation of patients. Therefore, it is hard for the doctor to adjust an examination pace.

Furthermore, in the case that a personal computer is used to display the time that the patient is received, the hospital can grasp the waiting situation of patients but the patients cannot know it.

### SUMMARY OF THE INVENTION

In consideration of the drawbacks of the related art, it is an object of the invention to provide a medical data managing system capable of causing staff in a hospital to simply grasp the waiting situation of patients, and furthermore, causing patients to easily grasp the waiting situation of the patients. with a simple operation.

In order to solve the aforesaid object, the invention is characterized by having the following arrangement.
(1) A medical data managing system comprises:
   recording means for recording patient data including identification data on a patient and inspection data;
   a display;
   reception input means for inputting reception data on the patient; and
   control means for displaying, on the display, at least part of the patient data and at least one of reception time and an elapse time from the reception time with linking each other.
(2) The medical data managing system according to (1) further comprising state input means for inputting a patient state representing at one of least received, inspected and examined,
   wherein the control means displays, on the display, at least part of the inputted patient state with linking to at least part of the patient data.
(3) The medical data managing system according to (2), wherein
   the state input means inputs a patient state as the received, and
   the control means displays, on the display, at least one of the reception time and the elapse time when the received is inputted.
(4) The medical data managing system according to any one of (1) to (3), wherein the reception input means includes reading means for reading and inputting the identification data on the patient recorded in a recording medium.
(5) The medical data managing system according to (4), wherein the control means displays, on the display, received as the patient state with linking to at least part of a patient data when the identification data on the patient is inputted as the reception data by the reading means.
(6) The medical data managing system according to any one of (1) to (5) further comprising image input means for inputting an image of a waiting room,
   wherein the control means displays the image of the waiting room on the display.
(7) The medical data managing system according to any one of (1) to (6), wherein the display includes at least one of a first display put in a consulting room and a second display put in a waiting room.
(8) A medical data managing system comprises:
   recording means for recording patient data including identification data on a patient and inspection data;
   a display;
   state input means for inputting a patient state representing at least one of received, inspected and examined; and
   control means for displaying, on the display, at least part of the patient data and the inputted patient status with linking each other.
(9) The medical data managing system according to (8), wherein
   the state input means includes reading means for reading and inputting the identification data on the patient recorded in a recording medium, and
   the control means displays the received as the patient status when the identification data on the patient is inputted by the reading means.
(10) The medical data managing system according to (8) or (9) further comprising image input means for inputting an image of a waiting room,
   wherein the control means displays the image of the waiting room on the display.
(11) The medical data managing system according to any one of (8) to (10), wherein the display includes at least one of a first display put in a consulting room and a second display put in a waiting room.
(12) A medical data managing system comprises:
   recording means for recording patient data including identification data on a patient and inspection data;
   a display;
   input means for inputting an image of a waiting room;
   control means for displaying, on the display, at least part of the patient data and the image of the waiting room.

The present disclosure relates to the subject matter contained in Japanese patent application No. 2000-240655 (filed on August 3, 2000), which is expressly incorporated herein by reference in its entirety.

### Brief Description of the Drawings

Fig. 1 is a schematic view showing the structure of a medical data managing system according to an embodiment of the invention,
Fig. 2 is a view showing a patient list screen that is one of screens when a medical data managing software is used,
Fig. 3 is a view showing a registration screen that is one of the screens displayed when the medical data managing software is used,
Fig. 4 is a view showing an image input screen that is one of the screens displayed when the medical data managing software is used,
Fig. 5 is a view showing an image storage screen that is one of the screens displayedwhen the medical datamanaging software is used,
Fig. 6 is a view showing a state in which an image window is displayed on the patient list screen,
Fig. 7 is a view showing a screen of a display put in a waiting room, and
Fig. 8 is a view showing a state in which the image of the image window is changed from normal display to enlarged display.

### DETAILED DESCRIPTION OF PREFERRED EMBODIMENT

An embodiment of the invention will be described with reference to the accompanying drawings.

### <Basic structure of the system>

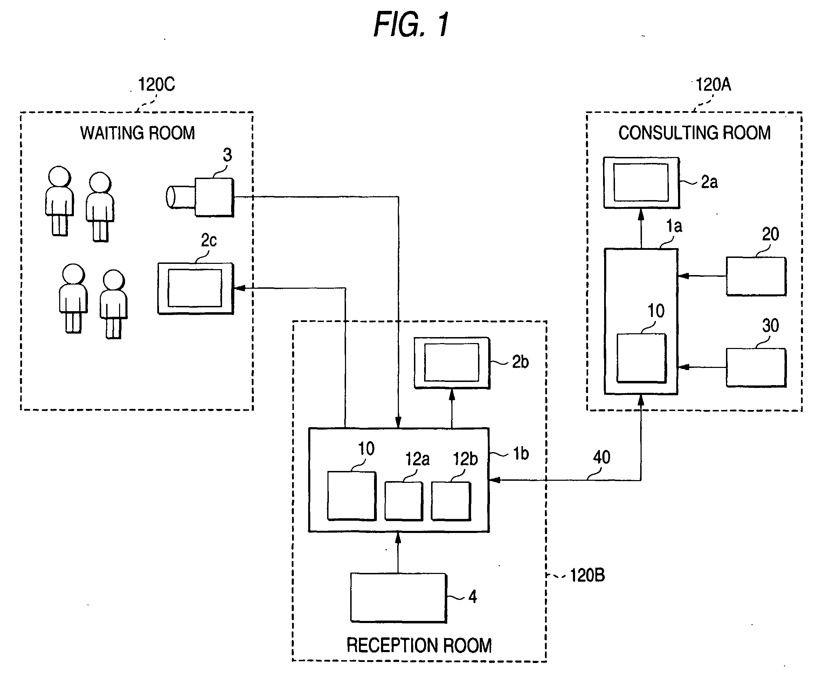

Fig. 1 shows a structure of a medical data managing system for collectively managing and processing patient data, such as image data, inspection (measured) data, medical examination data on others obtained by a medical apparatus. An ophthalmologic apparatus is usedas the medical apparatus in the present embodiment.

Personal computers 1a and 1b for managing patient data are put in a consulting room 120a and a reception room 120b. A medical data managing software 10 is recorded (installed) in nonvolatile memories (not shown) of the personal computers 1a and 1b. By starting up the software 10, a system can be used. In the embodiment, the personal computer 1b provided in the reception room 120b is used as a server for recording the patient data and the personal computer 1a provided in the consulting room 120a is used as a terminal. The personal computer 1a and the personal computer 1b are connected to each other through a LAN cable and various data stored on the server side (personal computer 1b) can be managed on the terminal side (personal computer 1a).

The medical apparatus (a fundus camera 20 and a slit lamp 30 in the embodiment) is connected to the personal computer 1a. An imaging system such as a CCD camera, a digital camera (not shown) or the like for capturing (obtaining) images is preliminarily provided to each of the fundus camera 20 and the slit lamp 30, so that image data obtained by each imaging system is inputted into the personal computer 1a, and the personal computer 1b via the input section 5.

Reference numerals 2a and 2b designate displays for which a general purposed display, such as a LCD or CRT monitor, can be used. The displays 2a and 2b are connected to the personal computers 1a and 1b, respectively. A display 2c for displaying waiting situation information of the patients is put in the waiting room for waiting the medical examination, and is connected to the personal computer 1b.

Reference numeral 3 denotes a CCD camera which is connected to the personal computer 1b and provided in a corner of the waiting room 120c to capture the whole waiting room. The image of the waiting room 120c is displayed on displays 2a and 2b by using the software 10. This will be described below in detail.

Reference numeral 4 denotes a card reader for reading a hospital identification card (not shown) which records personal data such as code (number) for recognizing a patient. By passing the hospital identification card through the card reader 4, a patient is received. While the card reader is used in the embodiment, it is not restricted but a device capable of carrying out a work for receiving the patient may be used. For example, in the case that personal data are bar code, a device for reading the bar code may be used. Moreover, the receiving work may be carried out through manual input by using a keyboard even if such a device is not used.

### <Structure of the medical data managing software>

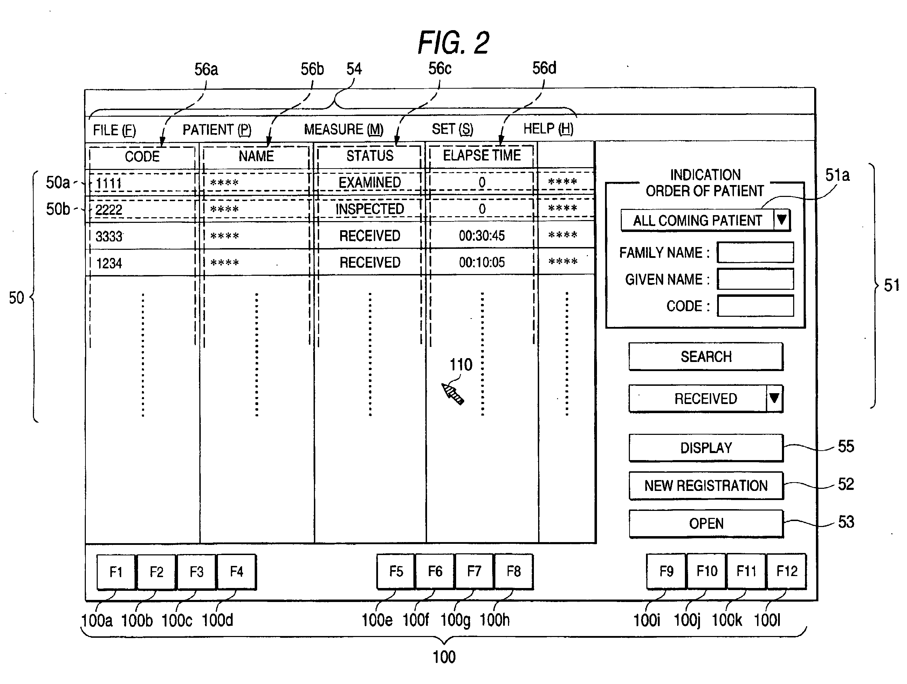

when the software 10 is started up, an operation screen for managing the patient data is displayed on the display 2a (2b). The operation screen is mainly made up of four processing screens. The operation screens are respectively shown in Figs. 2 to 5, and each of them will be explained.

### (Patient list screen)

When the software 10 is started up, a patient list screen is first displayed on the display 2a (2b) (see Fig. 2). This screen serves as a main screen with which indication of a list of already registered patients, search for a patient, and receipt (registration) of a new patient, and the like can be executed. The list of registered patients is indicated in order as a patient 50a, a patient 50b .... in a display column 50.

For each of the registered patients, a respective patient folder is prepare and the patient data is recorded in nonvolatile memories (not shown) of the personal computer 1b. In the present embodiment, a folder 12a for the patient 50a and a folder 12b for the patient 50b have been prepared, respectively.

At the right side of the screen, a vertical row (a column) is provided, which includes a button (icon) group 51 for searching a registered patient, a registration button (icon) 52 for new registration, an open (disclosure) button (icon) 53 for retrieving details of individual patient data, and a display button (icon) 55 for displaying the image from the camera 3 on the screen. To use any of these buttons (icons), a cursor mark 110 shown on the display 2a (2b) is moved onto a desired button (icon) with using the mouse (not shown), and then a button on the mouse is clicked.

A button group 51 includes a display condition selection button (icon) 51a for changing display conditions in a display column 50. By using (clicking) a pull-down button (icon) on the right endof the button 51a, the display conditions can be selected.

The display conditions can be selected from "all patients" for displaying all registered patients, "received" for displaying only received patients (unexamined patients), "all coming patients" for displaying coming patients, "inspected" for displaying only tested (inspected) patients and "examined" for displaying only examined patients. The reference numeral 51b denotes a status button for specifying the status of a patient (received, inspected, examined and finished).

An item column for patient data displayed on the display column 50 includes a code column 56a indicating a registration number (code number) of a patient, a name column 56b indicating a name of a patient, a status column 56c indicating the status of a patient (received, inspected, examined, finished), and an elapsed time column 56d indicating a time elapsed after a patient is received.

While the elapsed time column 56d displays the time elapsed after the reception time in the embodiment, it is preferable that such information as to indicate the waiting time of a patient, for example, a reception time should be displayed.

At the lower part of the screen, shortcut button (icon) group 100 is provided so that 12 pieces of shortcut buttons (icons) 100-a to 100-l can be used. The shortcut buttons (icons) provided in this embodiment include a button (icon) 100a for movement to the main screen, a button (icon) 100b for displaying the patient list, abutton (icon) 100c for inputting image data from the fundus camera 20, a button (icon) 100d for inputting image data from the slit lamp 30, abutton (icon) 100e for displaying, in a thumbnail fashion, all image data stored in the Queue folder (not shown), and a button (icons) 100f for outputting selected image data using the printer (not shown) . In this embodiment, no task is assigned to the remaining buttons (icons) 100-g to 100-l.

At the upper part of the screen, a menu bar 54 is displayed, which contains items of "File", "Patient", "Measure", "Set", and "Help", and main functions (such as switch of a displayed screen) may be selected and used via this menu bar 54.

### (Registration screen)

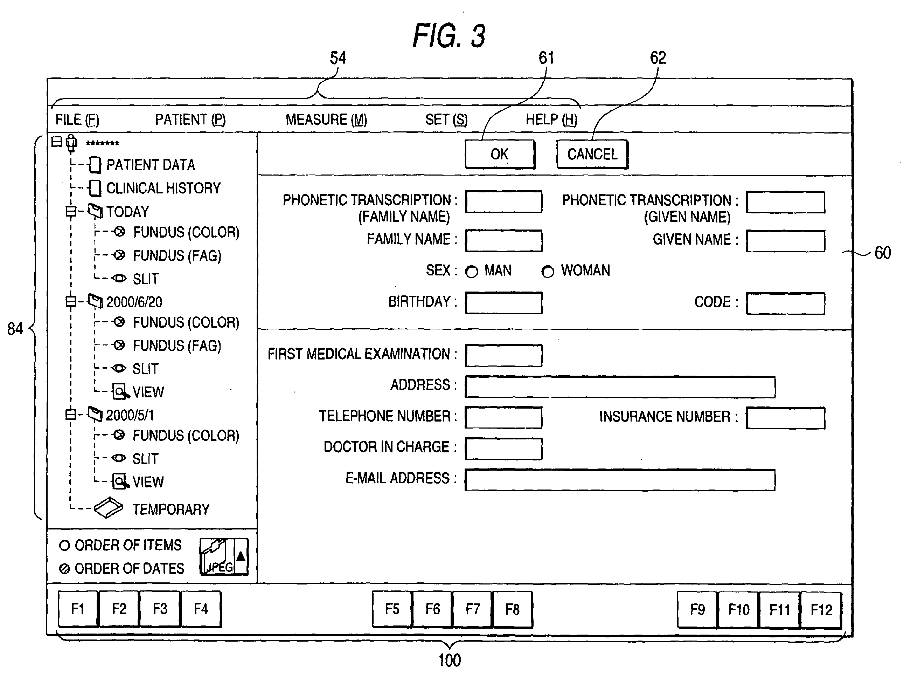

Fig. 3 shows a screen with which a new patient is registered, or registered patient data is displayed. This registration screen can be displayed upon operation of the button 52 or the button 53 shown in Fig. 2. At the left side of the registration screen, buttons (icons) are displayed in a hierarchical structure view (tree view 84), which respectively identify collected medical data such as image data obtained till now and measured data, and their stored contents or conditions are visually grasped.

A patient data registration screen 60 is used to register a new patient. Input items of the patient data are "Name", "Phonetic transcription", "Sex", "Birthday", "Code", "Day of first medical examination", "Address", "Tel. No." and the like. Reference numeral 61 designates an OK button (icon) for executing registration, and 62 is a cancel button (icon) for canceling registration. By performing a new registration, patient folders are prepared (recorded) in the nonvolatile memories of the personal computer 1b correspondingly to the number of the new registration.

### (Image input (capture) screen)

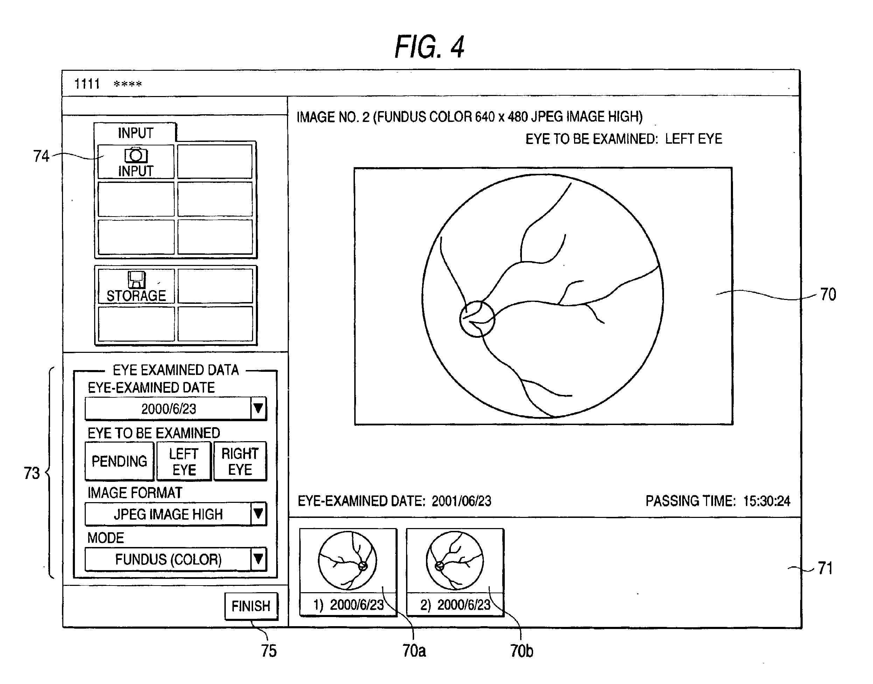

Fig. 4 shows an image input (capture) screen with which patient's eye image data obtained by the fundus camera 20 or the slit lamp 30 is input (captured) into the PC 1b (1a).

Reference numeral 70 designates a main image box, in which a latest inputted image is shown largely. 71 is a thumbnail indication box, in which the inputted images are displayed sequentially as thumbnail images 70a, 70b.

Reference numeral 73 designates a set button (icon) group for setting data of eye examination, such as date of eye examination, designation of right or left eye, an image format and image capture mode, with respect to the inputted image.

The image of patient's eye is inputted from the ophthalmologic apparatus (the fundus camera 20 or the slit lamp 30) by clicking an input button (icon) 74. The input operation for the image data may be carried out using a joystick or a foot switch (both not shown) provided in the ophthalmologic apparatus other than the use of the input button 74. Reference numeral 75 is a finish button (icon) for finishing the image input operation, and returning to the latest previous operation screen.

### (Image storage screen)

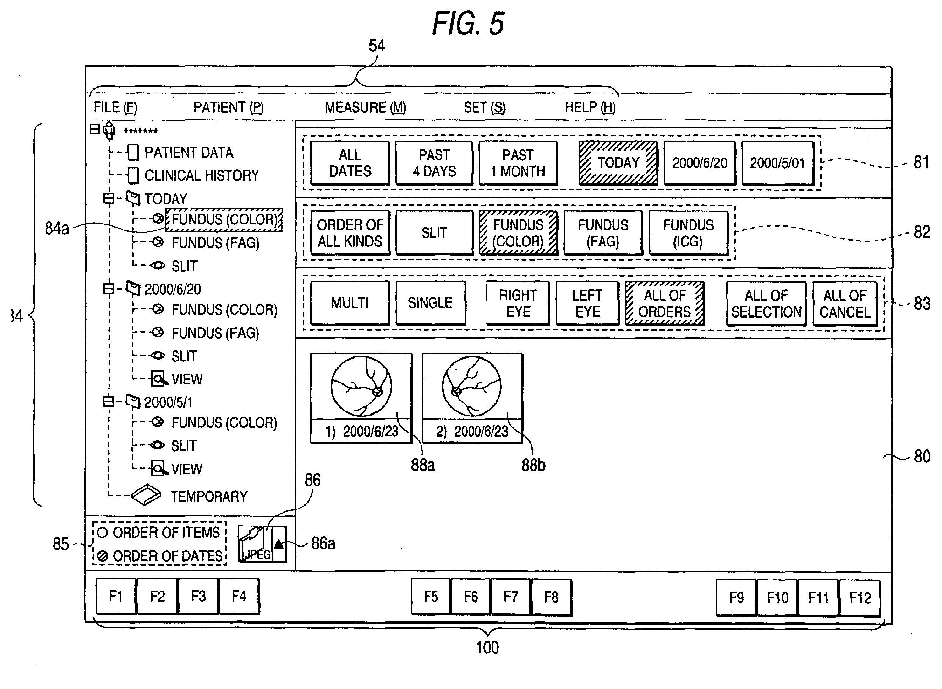

Fig. 5 shows an image storage screen in which the image data stored in the folder is displayed.

At the left side of the screen, various kinds of data stored in the folder are displayed in a hierarchical structure view (tree view 84) in date order or in item order (a fundus image, a slit image, etc.). A radio button 85 may be used to alter the hierarchical structure.

By selecting a button (icon) (for example, a fundus image button (icon) 84a) displayed in the tree view 84, the image data corresponding to the selected button (icon) is displayed as thumbnails 88a, 88b in the screen 80.

A plurality of buttons (icons), set in a date selection tool bar 81 for selecting a date of an image to be displayed, a kind selection tool bar 82 for selecting a kind of an image to be displayed, and a control tool bar 83 for designating a displaying condition such as a left or right eye, maybe selectively used to select a thumbnail image to be displayed on the display screen 80.

Reference numeral 86 is an output button (icon) for outputting the thumbnail image displayed on the display screen 80 (for usage other than the image data to be used in the software 10). Beside the output button 86, a button (icon) 86a for a pulling down menu is provided, and by clicking the button 86a, the output type (form) may be set variously.

The operation of the system having the above structure will be described.

First of all, the software 10 in the personal computers 1a and 1b is started up and the "all coming patients" is selected through the button 51a, so that a main screen (patient list screen) is displayed (see Fig. 2). A hospital identification card brought by a patient is passed through the card reader 4 in the reception room 120b so that the reception of the patient in the software 10 is completed. When the reception is completed, each data on the code, name and state (status) of the patient are displayed on each item column of the display column 50.

"received" is displayed in a status column 56c for the patient passing the hospital identification card through the card reader 4. Moreover, an elapsed time (hour, minute, second) based on a time (reception) that the hospital identification card is passed through the card reader 4 is displayed on an elapsed time column 56d. The elapsed time is measured by utilizing a clock function in the personal computer 1a (1b) and is updated at any time. By carrying out the same work for a next patient, data on the patient (code, name, state, elapsed time) are displayed on the screen 50 in order of reception.

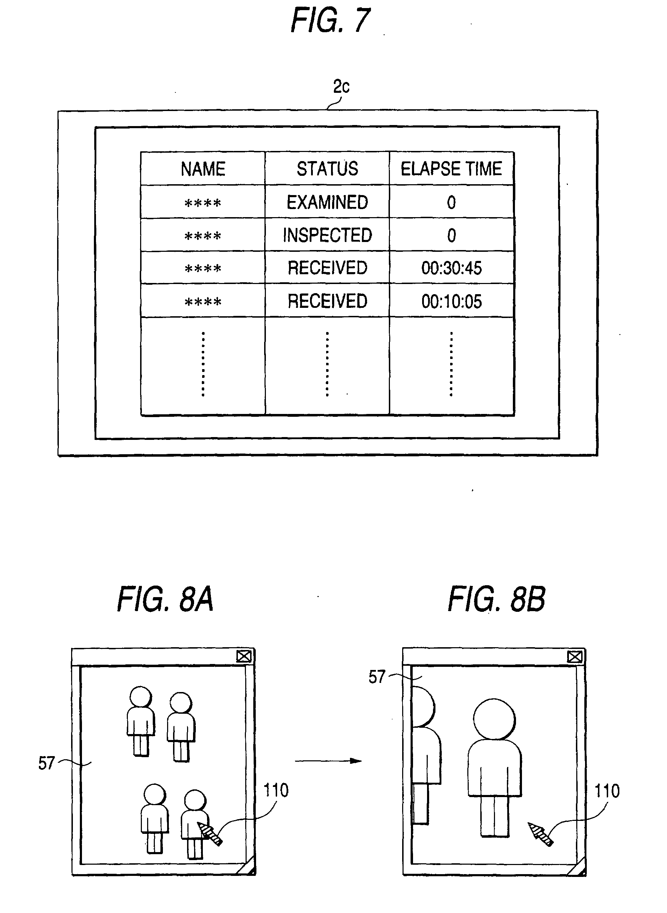

On the other hand, a display 2c put in the waiting room 120c always displays only the name of a patient, the state of the patient (received, inspected, examined) and the elapsed time based on a program of the software 10 (see Fig. 7). Consequently, the patient waiting in the waiting room can see the display 2c to easily confirm the numbers of inspected and examined patients and the number of patients waiting for an examination before a certain patient.

While the name of a patient is displayed on the display 2c in the embodiment, a reference number for the order of passage can be given to the patient after passing the hospital identification card and displayed on the display 2c in place of the name of the patient in order to protect privacy, for example.

The patient called for an inspection enters the consulting room 120a and is inspected by using a fundus camera 20 and a slit lamp 30. In the inspection, a work is carried out on the image input screen (see Fig. 4) and the image storage screen (see Fig. 5) to input image data on the patient to the personal computer 1b. When the inspection is finished, a doctor (or a receptionist) selects the inspected patient through the screen 50 and sets the state of the patient to the "inspected" by using the button 51b.

An examination is carried out for the inspected patient based on the image data inputted during the inspection. Similarly, the state of the examined patient is set to "examined" by using the button 51b. The elapsed time for the patient thus inspected and examined through the button 51b is displayed as 0 in the display column.

The software 10 in the embodiment is programmed such that the patient data displayed in the display column 50 is updated every one minute. If the state of the patient is changed on the personal computer 1a side, the state of the patient in the display column 50 which is displayed on the personal computer 1b side is also changed during the update of the display column 50. Accordingly, if the state of the patient is changed through one of the personal computers, the contents of the change are reflected in the state of the patient displayed on all the displays (2a, 2b, 2c) during the update.

For the patient who finishes the inspection and examination, the "finished" is selected through the button 51b. The patient for which the "finished" is selected is not displayed on the display 50 until the "all patients" is selected through the button 51a.

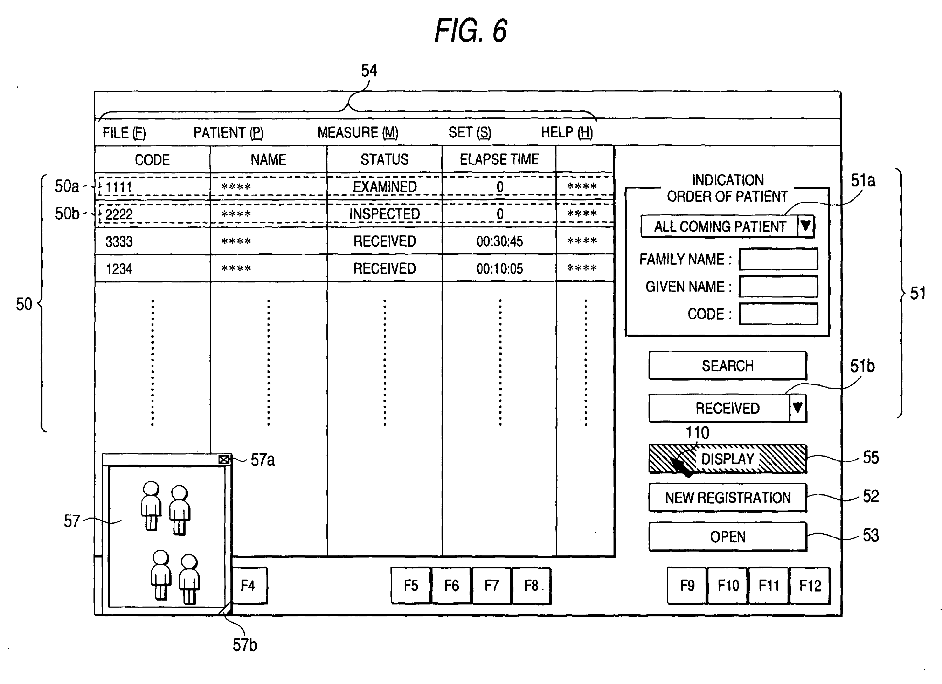

Moreover, programming is carried out such that an image window 57 appears in a top screen (first screen) on the display 2b when the button 55 on the main screen (see Fig. 2) is clicked in the personal computer 1b (see Fig. 6). The image of the waiting room 120c captured by the camera 3 is displayed on the window 57 in real time. Also in the personal computer 1a, the same operation is carried out so that the window 57 can be displayed, and the image of the waiting room 120c can be displayed in real time through the personal computer 1b. When the window 57 is thus displayed, the situation of the waiting room 120c can be grasped in another room.

Moreover, the software 10 is programmed so that the window 57 is always displayed on the top screen. Consequently, even if the operation screen of the software 10 is changed, the window 57 can always be seen on the first screen. To close the window 57, a button 57a is clicked or the button 55 is clicked again.

Furthermore, it is possible to optionally change the size of the window 57 to be displayed. In this case, it is preferable that a cursor 110 should be moved to a corner portion 57b by using a mouse and should be moved while dragging the corner portion 57b. By dragging and moving the corner portion 57b through the cursor 110, the size of the whole window 57 can be varied optionally without changing an aspect display ratio. Moreover, a portion other than the corner portion 57b of the window 57 can be moved to an optional portion in the screen by dragging.

While the situation of the waiting room 120C can be displayed on the window 57 while displaying the operation screen of the software 10 in the embodiment, an image in the window 57 may be further displayed largely.

Figs. 8A and 8B are schematic views showing the state in which the image of the window 57 is changed from normal display to enlarged display. As shown in Fig. 8A, any portion in the window 57 is designated (clicked) through the cursor 110. When any portion is designated through the cursor 110, the designated portion is largely displayed over the whole window 57 by a predetermined range (for example, 100 x 100 pixels) by setting the designated position as the center of the window 57 as shown in Fig. 8B. A zoom displaying method is preferably carried out in software (digital zoom).

Moreover, to obtain an enlarged image with a higher resolution, a CCD camera having an optical zoom function and driving system for turning in vertical and transverse directions may be prepared as the camera 3. When any portion in the window 57 is designated through the cursor 110, the camera 3 is driven and controlled in the vertical and transverse directions through the personal computer 1a (1b) such that the designated portion is set to be the center of the window 57. Furthermore, the optical zoom is carried out so that the enlarged display can be obtained on the window 57 with a predetermined magnification.

By providing such a zoom displaying function, the state of a specific patient can be observed in detail and a subsequent countermeasure can be taken easily.

Moreover, while the enlarged display is carried out by using the mouse through the cursor 110 displayed on the display, it is not restricted but the enlarged display can be designated through a keyboard or a joy stick, for example.

As described above, according to the invention, a doctor can simply grasp the situation of waiting patients in another room. Furthermore, the patients waiting in the waiting room can also grasp the waiting situation easily.

## Claims

1. A medical data managing system comprising;
recording means for recording patient data including identification data on a patient and inspection data;
a display;
reception input means for inputting reception data on the patient; and
control means for displaying, on the display, at least part of the patient data and at least one of reception time and an elapse time from the reception time with linking each other.

2. The medical data managing system according to claim 1 further comprising state input means for inputting a patient state representing at one of least received, inspected and examined,
wherein the control means displays, on the display, at least part of the inputted patient state with linking to at least part of the patient data.

3. The medical data managing system according to claim 2,
wherein
the state input means inputs a patient state as the received, and
the control means displays, on the display, at least one of the reception time and the elapse time when the received is inputted.

4. The medical data managing system according to any one of claims 1 to 3, wherein the reception input means includes reading means for reading and inputting the identification data on the patient recorded in a recording medium.

5. The medical data managing system according to claim 4, wherein the control means displays, on the display, received as the patient state with linking to at least part of a patient data when the identification data on the patient is inputted as the reception data by the reading means.

6. The medical data managing system according to any one of claims 1 to 5 further comprising image input means for inputting an image of a waiting room,
wherein the control means displays the image of the waiting room on the display.

7. The medical data managing system according to any one of claims 1 to 6, wherein the display includes at least one of a first display put in a consulting room and a second display put in a waiting room.

8. A medical data managing system comprising;
recording means for recording patient data including identification data on a patient and inspection data;
a display;
state input means for inputting a patient state representing at least one of received, inspected and examined; and
control means for displaying, on the display, at least part of the patient data and the inputted patient status with linking each other.

9. The medical data managing system according to claim 8,
wherein
the state input means includes reading means for reading and inputting the identification data on the patient recorded in a recording medium, and
the control means displays the received as the patient status when the identification data on the patient is inputted by the reading means.

10. The medical data managing system according to claim 8 or 9 further comprising image input means for inputting an image of a waiting room,
wherein the control means displays the image of the waiting room on the display.

11. The medical data managing system according to any one of claims 8 to 10, wherein the display includes at least one of a first display put in a consulting room and a second display put in a waiting room.

12. A medical data managing system comprising:
recording means for recording patient data including identification data on a patient and inspection data;
a display;
input means for inputting an image of a waiting room;
control means for displaying, on the display, at least part of the patient data and the image of the waiting room.
